# EUROPEAN PATENT APPLICATION

(11) **EP 2 033 958 A1**
(43) Date of publication of application: **11.03.2009**
(21) Application number: 07075774.5
(22) Date of filing: 07.09.2007
(51) Int. Cl.: C07D 307/46

(54) **Hydroxymethylfurfural ethers from sugars and di- and triols**

(71) Applicant: Furanix Technologies B.V, 1014 BV Amsterdam (NL)
(72) Inventor: Gruter, Gerardus Johannes Maria, 2106 BA Heemstede (NL)
(74) Representative: Kortekaas, Marcel C.J.A.

(57) **Abstract**

The current invention provides a method for the manufacture of an ether of 5-hydroxymethylfurfural by reacting a hexose-containing starting material or HMF with a C2 to C6 di- or triol other than 2-methyl-1,3-propanediol, in the presence of an acid catalyst.

## Description

### Technical Field

The present invention concerns a method for the manufacture of an ether of 5-hydroxymethylfurfural (5-(hydroxymethyl)-2-furaldehyde, or HMF) from biomass.

### Background Art

Fuel, fuel additives and various chemicals used in the petrochemical industry are derived from oil, gas and coal, all finite sources. Biomass, on the other hand, is considered a renewable source. Biomass is biological material (including biodegradable wastes) which can be used for the production of fuels or for industrial production of e.g. fibres, chemicals or heat. It excludes organic material which has been transformed by geological processes into substances such as coal or petroleum.

Production of biomass derived products for non-food applications is a growing industry. Biobased fuels are an example of an application with strong growing interest..

Biomass contains sugars (hexoses and pentoses) that may be converted into value added products. Current biofuel activities from sugars are mainly directed towards the fermentation of sucrose or glucose into ethanol or via complete breakdown via Syngas to synthetic liquid fuels. EP 0641 854 describes the use of fuel compositions comprising of hydrocarbons and/or vegetable oil derivatives containing at least one glycerol ether to reduce particulate matter emissions.

More recently, the acid catalysed reaction of fructose has been re-visited, creating HMF as an intermediate of great interest. Most processes investigated have the disadvantage that HMF is not very stable at the reaction conditions required for its formation. Fast removal from the water-phase containing the sugar starting material and the acid catalyst has been viewed as a solution for this problem. Researchers at the University of Wisconsin-Madison have developed a process to make HMF from fructose. HMF can be converted into monomers for plastics, petroleum or fuel extenders, or even into fuel itself. The process by prof. James Dumesic and co-workers first dehydrates the fructose in an aqueous phase with the use of an acid catalyst (hydrochloric acid or an acidic ion-exchange resin). Salt is added to salt-out the HMF into the extracting phase. The extracting phase uses an inert organic solvent that favors extraction of HMF from the aqueous phase. The two-phase process operates at high fructose concentrations (10 to 50 wt %), achieves high yields (80% HMF selectivity at 90% fructose conversion), and delivers HMF in a separation-friendly solvent ( DUMESIC, James A, et al. "Phase modifiers promote efficient production of Hydroxymethylfurfural from fructose" . Science. 30 juni 2006, vol.312, no.5782, p.1933-1937). Although the HMF yields from this process are interesting, the multi-solvent process has cost-disadvantages due to the relatively complex plant design and because of the less than ideal yields when cheaper and less reactive hexoses than fructose, such as glucose or sucrose, are used as a starting material. HMF is a solid at room temperature which has to be converted in subsequent steps to useful products. Dumesic has reported an integrated hydrogenolysis process step to convert HMF into dimethylfuran (DMF), which is assumed to be an interesting gasoline additive.

In WO 2006/063220 a method is provided for converting fructose into 5- ethoxymethylfurfural (EMF) at 60 °C, using an acid catalyst either in batch during 24 hours or continuously via column elution during 17 hours. Applications of EMF were not discussed.

Also in copending patent application PCT/EP2007/002145 the manufacture of HMF ethers are described, including the use of such ethers as fuel or fuel additive. Indeed, both the methyl ether and the ethyl ether (methoxymethylfurfural, or MMF; ethoxyethylfurfural or EMF) were prepared and tested. The invention of the copending patent application, however, was limited to the use of primary aliphatic alcohols, and preferably primary C1-C5 alcohols. Use of secondary and tertiary alcohols was not considered, whereas the only example of a branched primary alcohol was considered, was a diol ("2-hydroxymethyl-propanol", which is 2-methyl-1,3-propanediol). Although MMF and EMF are useful as fuel or fuel additive, the inventors found that the ethers leave room for improvement, in particular when used in higher concentration blends with fuels such as gasoline, kerosene, diesel, biodiesel or green diesel. The inventors have therefore set out to overcome this shortfall.

Surprisingly, the inventors have found that ethers of HMF obtained from di- or triols have superior blending properties compared to ethers obtained from methanol or ethanol analogs.

The ethers of HMF with these alcohols may be produced in a reasonable yield from hexose containing feedstock or from HMF, with reduced levels of by-product formation and in a manner that does not require cumbersome process measures (such as 2-phase systems) or lengthy process times.

### Disclosure of Invention

Accordingly, the current invention provides a method for the manufacture of an ether of 5-hydroxymethylfurfural by reacting a hexose-containing starting material or HMF with a C2 to C6 di- or triol other than 2-methyl-1,3-propanediol, in the presence of an acid catalyst.

When the reaction product of the above method is used as such or when it is used as an intermediate for a subsequent conversion, the selectivity of the reaction is preferably high as the product is preferably pure. However, when the reaction product of the above method is used as a fuel, a fuel additive or as a fuel or a fuel additive intermediate, the reaction product does not necessarily need to be pure. Indeed, in the preparation of fuel and fuel additives from biomass, which in itself is a mixture of various monosaccharides, disaccharides and polysaccharides, the reaction product may contain non-interfering components such as levulinic acid derivatives and/or derivatives of pentoses and the like. For ease of reference, however, the method and the reaction product are described in terms of the reaction of a hexose-containing starting material, resulting in an ether of HMF. Also within the scope of the invention is the reaction of HMF with the branched alcohol, since HMF is believed to be produced as intermediate from the hexose-containing starting material.

The current invention also provides for the use of the reaction product made according to the present invention as fuel or as fuel additive. Fuels for blending with the product of the present invention include but are not limited to gasoline and gasoline-ethanol blends, kerosene, diesel, biodiesel (all renewable fuels combustible in a diesel engine), Fischer-Tropsch liquids (for example obtained from GTL, CTL or BTL gas-to-liquids/coal-to-liquids/biomass to liquids proceses), diesel-biodiesel blends and green diesel and blends of diesel and/or biodiesel with green diesel (green diesel is a hydrocarbon obtained by hydrotreating biomass derived oils, fats, greases or pyrolysis oil; see for example the UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085)). Fuels for blending with the product of the present invention may also include one or more other furanics, wherein the expression furanics is used to include all derivatives of furan and tetrahydrofuran. The invention also provides a fuel composition comprising a fuel element as described above and the reaction product made according to the present invention.

### Mode(s) for Carrying Out the Invention

Biomass resources are well known. The components of interest in biomass are the mono-, di- or polysaccharides (hereinafter referred to as hexose containing starting material). Suitable 6-carbon monosaccharides include but are not limited to fructose, glucose, galactose, mannose and their oxidized, reduced, etherified, esterified and amidated derivatives, e.g. aldonic acid or alditol, with glucose being the most abundant, the most economic and therefore the most preferred monosaccharide, albeit less reactive than fructose. On the other hand, the current inventors have also succeeded to convert sucrose, which is also available in great abundance. Other disaccharides that may be used include maltose, cellobiose and lactose. The polysaccharides that may be used include cellulose, inulin (a polyfructan), starch (a polyglucan), and hemi-cellulose. The polysaccharides and disaccharides are converted into their monosaccharide component(s) and dehydrated during the manufacture of the 5-HMF ether.

The di- or triol used in the method of the current invention preferably bears two or three hydroxyl groups, which may be in a primary, secondary or even tertiary position. 2-Methyl-1,3-propanediol is preferably excluded from the scope of the present application. The alcohol may comprise from 2 to 6 carbon atoms, preferably from 2 or 3 carbon atoms. Examples include 1,2-ethanediol (ethylene glycol); 1,2-propanediol, 1,3-propanediol; 1,2,3-propanetriol (glycerol); and 1,6-hexanediol. Also cycloaliphatic diols and triols may be used, including such compounds containing oxygen in the aliphatic ring. Examples include dihydroxymethylfuran and dihydroxymethyl tetrahydrofuran, which both may be derived from biomass.

Also blends of alcohols may be used, e.g., of glycol and glycerol. The current method thus provides an excellent high value outlet for glycerol that is "contaminated" with ethylene glycol and/or propylene glycol.

The amount of di- or triol used during the manufacture of the HMF ether is preferably at least equimolar on the hexose content of the feedstock, but typically is used in much greater excess. Indeed, the alcohol (such as ethylene glycol) may be used as solvent or co-solvent. In such a case, a sufficient amount of alcohol is present to form the HMF ether.

The acid catalyst in the method of the present invention can be selected from amongst (halogenated) organic acids, inorganic acids, Lewis acids, ion exchange resins and zeolites or combinations and/or mixtures thereof. It may be a homogeneous catalyst, but heterogeneous catalysts are preferred for purification reasons. The HMF ethers can be produced with a protonic, Brønsted or, alternatively, a Lewis acid or with catalysts that have more than one of these acidic functionalities.

The protonic acid may be organic or inorganic. For instance, the organic acid can be selected from amongst oxalic acid, levulinic acid, maleic acid, trifluoro acetic acid (triflic acid), methansulphonic acid or para-toluenesulphonic acid. Alternatively, the inorganic acid can be selected from amongst (poly)phosphoric acid, sulphuric acid, hydrochloric acid, hydrobromic acid, nitric acid, hydroiodic acid, optionally generated in situ.

Certain salts may be used as catalyst, wherein the salt can be any one or more of (NH₄)₂SO₄/SO₃, ammonium phosphate, pyridinium chloride, triethylamine phosphate, pyridinium salts, pyridinium phosphate, pyridinium hydrochloride/hydrobromide/perbromate, DMAP, aluminium salts, Th and Zr ions, zirconium phosphate, Sc and lanthanide ions such as Sm and Y as their acetate or trifluoroactate (triflate) salt, Cr-, Al-, Ti-, Ca-, In-ions, ZrOCl₂, VO(SO₄)₂, TiO₂, V-porphyrine, Zr-, Cr-, Ti-porphyrine.

Lewis acids selected as dehydration catalyst can be any one of ZnCl₂, AlCl₃, BF₃.

Ion exchange resins can be suitable dehydration catalysts. Examples include Amberlite™ and Amberlyst^{™}, Diaion™ and Levatit™. Other solid catalyst that may be used include natural clay minerals, zeolites, supported acids such as silica impregnated with mineral acids, heat treated charcoal, metal oxides, metal sulfides, metal salts and mixed oxides and mixtures thereof.

An overview of catalysts that may be used in the method of the current invention may be found in Table 1 of the review article prepared by Mr. Lewkowski: "Synthesis, chemistry and applications of 5-hydroxymethylfurfural and its derivatives" Arkivoc. 2001, p.17-54.

The amount of catalyst may vary, depending on the selection of catalyst or catalyst mixture. For instance, the catalyst can be added to the reaction mixture in an amount varying from 0.01 to 40 mole % drawn on the hexose content of the biomass resource, preferably from 0.1 to 30 mole %, more preferably from 1 to 20 mole %.

In the preferred embodiment, the catalyst is a heterogeneous catalyst.

The temperature at which the reaction is performed may vary, but in general it is preferred that the reaction is carried out at a temperature from 50 to 300 degrees Celsius, preferably from 125 to 250 degrees Celsius, more preferably from 150 to 225 degrees Celsius. In general, temperatures higher than 300 are less preferred as the selectivity of the reaction reduces and as many by-products occur, inter alia caramelisation of the sugar. Performing the reaction below the lowest temperature is also less preferable because of the low reaction rate.

The HMF or hexose-containing starting material is typically dissolved or suspended in a solvent which can also be the branched alcohol reactant, in order to facilitate the reaction. The solvent system may be selected from one or more of the group consisting of water, sulfoxides, preferably DMSO, ketones, preferably methyl ethylketone, methylisobutylketone and acetone, ethylene glycol ethers, preferably diethyleneglycol dimethyl ether (diglyme). Also so-called ionic liquids may be used. The latter refers to a class of inert ionic compounds with a low melting point, which may therefore be used as solvent. Examples thereof include e.g., 1-H-3-methyl imidazolium chloride, discussed in "Dehydration of fructose and sucrose into 5-hydroxymethylfurfural in the presence of 1-H-3-methyl imidazolium chloride acting both as solvent and catalyst", by Claude Moreau et al, Journal of Molecular Catalysis A: Chemical 253 (2006) 165-169.

Basically a sufficient amount of solvent is preferable present to dissolve or suspend the starting material and to limit undesired side-reactions.

The method of the current invention may be carried out in a batch process or in a continuous process, with or without recycle of (part of) the product stream to control the reaction temperature (recycle via a heat exchanger). For instance, the method of the invention can be performed in a continuous flow process. In such method, homogenous catalysts may be used and the residence time of the reactants in the flow process is between 0.1 second and 10 hours, preferably from 1 second to 1 hours, more preferably from 5 seconds to 20 minutes.

Alternatively, the continuous flow process may be a fixed bed continuous flow process or a reactive (catalytic) distillation process with a heterogeneous acid catalyst. To initiate or regenerate the heterogeneous acid catalyst or to improve performance, an inorganic or organic acid may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100 min⁻¹.

The above process results in a stable HMF ether, which can then be used as such or be converted into a further derivative before being used as fuel and/or as fuel additive. The inventors are of the opinion that some of the products prepared by the method of the current invention are actually new. Thus, the monoethers and diethers made with ethylene glycol, 1,2-propylene glycol, 1,3-propylene glycol, glycerol, dihydroxymethylfuran or dihydroxymethyl THF as alcohol, are new and are excellent fuel components or fuel additives. Since these alcohols may be made from biomass, this might open a class of products that are fully biomass-derived. Accordingly, these new ethers are claimed as well.

The HMF ethers of the invention can also be used as or can be converted to compounds that can be used as solvent, as monomer in a polymerization (such as 2,5-furan dicarboxylic acid or FDCA), as fine chemical or pharmaceutical intermediate, or in other applications. Oxidation of the HMF ethers using an appropriate catalyst under appropriate conditions such as for example described for p-xylene with a NHPI/Co(OAc)₂/MnOAc)₂ catalyst system in Adv. Synth. Catal. 2001, 343, 220-225 or such as described for HMF with a Pt/C catalyst system at pH < 8 in EP 0 356 703 or or such as described for HMF with a Pt/C catalyst system at pH > 7 in FR 2 669 634, all with air as an oxidant, resulted in the formation of 2,5-furan dicarboxylic acid (FDCA).

The invention further concerns the use of the HMF ethers prepared by the method of the current invention as fuel and/or as fuel additive. Of particular interest is the use of the ethers in diesel, biodiesel or "green diesel", given its (much) greater solubility therein than ethanol. Conventional additives and blending agents for diesel fuel may be present in the fuel compositions of this invention in addition to the above mentioned fuel components. For example, the fuels of this invention may contain conventional quantities of conventional additives such as cetane improvers, friction modifiers, detergents, antioxidants and heat stabilizers, for example. Especially preferred diesel fuel formulations of the invention comprise diesel fuel hydrocarbons and HMF ether as above described together with peroxidic or nitrate cetane improvers such as ditertiary butyl peroxide, amyl nitrate and ethyl hexyl nitrate for example.

Examples are enclosed to illustrate the method of the current invention and the suitability of the products prepared therefrom as fuel. The examples are not meant to limit the scope of the invention.

### Example 1. 5-(hydroxymethyl)furfural ethylene glycol ether (5-(2-hydroxyethoxymethyl)furfural) formation

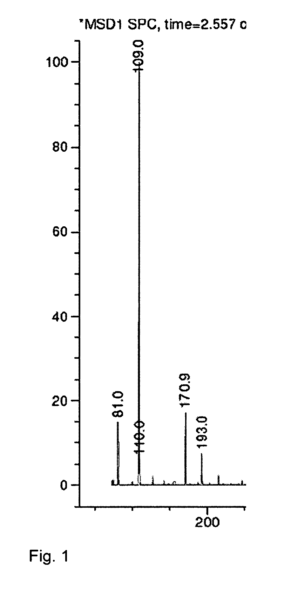

In a 7.5 mL batch reactor, equipped with on-line transmission turbidity measurement, 0.82 mmol fructose was added to 0.8 mL ethylene glycol containing 0.5 wt% sulphuric acid. The reaction mixture was heated at 85 °C until all fructose was dissolved (approx. 10 minutes). Two main furanics peaks were detected using HPLC with UV detection and their identity was shown to be HMF and 2-(hydroxyethyl)oxymethyl)furfural by LC-MS (Cl). See fig. 1.

### References

- DUMESIC, James A, et al. "Phase modifiers promote efficient production of Hydroxymethylfurfural from fructose". Science. 30 June 2006, vol.312, no.5782, p.1933-1937.
- WO 2006/063220
- Chapter 15 of Advanced Organic Chemistry, by Jerry March, and in particular under reaction 5-4. (3rd ed., © 1985 by John Wiley & Sons, pp. 684-685).
- LEWKOWSKI, Jaroslaw. Synthesis, chemistry and applications of 5-hydroxymethylfurfural and its derivatives. Arkivoc. 2001, p.17-54.
- MOREAU, Claude, et al. "Dehydration of fructose and sucrose into 5-hydroxymethylfurfural in the presence of 1-H-3-methyl imidazolium chloride acting both as solvent and catalyst", Journal of Molecular Catalysis A: Chemical 253 (2006) p. 165-169.
- EP 0641 854
- UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085))
- Adv. Synth. Catal. 2001, 343, 220-225
- EP 0 356 703
- FR 2 669 634

## Claims

1. Method for the manufacture of an ether of 5-hydroxymethylfurfural by reacting a hexose-containing starting material with a C2-C6 di- or triol, other than 2-methyl-1,3-propanediol, in the presence of an acid catalyst.

2. Method according to claim 1, wherein the C2-C6 di- or triol is selected from the group comprising 1,2-ethanediol, 1,2-propanediol, 1,3-propanediol; 1,2,3-propanetriol, 1,6-hexanediol, di(hydroxymethyl)furan and di(hydroxymethyl) tetrahydrofuran

3. Method according to claim 1 or 2, wherein the acid catalyst is selected from the group consisting of homogeneous or heterogeneous acids selected from solid organic acids, inorganic acids, salts, Lewis acids, ion exchange resins, zeolites or mixtures and/or combinations thereof.

4. Method according to claim 1, wherein the acid is a solid Brønsted acid.

5. Method according to claim 1, wherein the acid is a solid Lewis acid.

6. Method according to any one of the claims 1 to 5, wherein the reaction is performed at a temperature from 50 to 300 degrees Celsius, preferably from 125 to 250, more preferably from 150 to 225 degrees Celsius.

7. Method according to any one of the claims 1 to 6, wherein a hexose-containing starting material is used and wherein the hexose starting material is selected from the group of
• starch, amylose, galactose, cellulose, hemi-cellulose,
• glucose-containing disaccharides such as sucrose, maltose, cellobiose, lactose, preferably glucose-containing disaccharides, more preferably sucrose,
• glucose or fructose.

8. Method according to any one of the claims 1 to 6, wherein the starting material is 5-(hydroxymethyl)furfural.

9. Method according to any one of the claims 1 to 6, wherein the starting material comprises glucose, fructose, galactose and mannose and their oxidized (aldonic acid) or reduced (alditol) derivatives or mixtures thereof.

10. Method according to any one of the claims 1 to 6 wherein the starting material is an esterified, etherified monosaccharide or an amido sugar.

11. Method according to any one of the claims 1 to 10, wherein the solvent or solvents are selected form the group consisting of water, sulfoxides, preferably DMSO, ketones, preferably methyl ethylketone, ionic liquids, methylisobutylketone and/or acetone, esters, ethers, preferably ethylene glycol ethers, more preferably diethyleneglycol dimethyl ether (diglyme) or the reactant olefin and mixtures thereof.

12. Method according to any one of the claims 1 to 11, wherein the method is performed in a continuous flow process.

13. Method according to claim 12, wherein the residence time in the flow process is between 0.1 second and 10 hours, preferably from 1 second to 1 hours, more preferably from 5 seconds to 20 minutes.

14. Method according to claim 13, wherein the continuous flow process is a fixed bed continuous flow process.

15. Method according to claim 14, wherein the fixed bed comprises a heterogeneous acid catalyst.

16. Method according to claim 15, wherein the continuous flow process is a reactive distillation or a catalytic distillation process.

17. Method according to claim 16, wherein in addition to a heterogeneous acid catalyst, an inorganic or organic acid catalyst is added to the feed of the fixed bed or catalytic distillation continuous flow process.

18. Method according to claim 14-17, wherein the LHSV is from 1 to 1000, preferably from 5 to 500, more preferably from 10 to 250 and most preferably from 25 to 100.

19. Use of the ether produced by the method of any one of claims 1-18 as a solvent, as a pharmaceutical intermediate or as an olefinically unsaturated monomer from an olefinically unsaturated di- or triol or as an intermediate for the production of a polyester monomer.

20. Use of the ether produced by the method of any one of claims 1-18 as fuel or fuel additive.

21. A fuel or fuel composition comprising the ether produced by the method of any one of claims 1-18 as fuel component, optionally blended with one or more of gasoline and gasoline-ethanol blends, kerosene, diesel, biodiesel, Fischer-Tropsch liquids, diesel-biodiesel blends and green diesel and blends of diesel and/or biodiesel with green diesel and other furanics.

22. 5-(hydroxymethyl)furfural 2-hydroxyethyl ether (ether of 1,2-ethanediol and HMF)

23. bis 5-(hydroxymethyl)furfural 1,2-ethane-diyl di-ether (di-ether of 1,2-ethanediol and 2 equivalents of HMF)

24. 5-(hydroxymethyl)furfural 2-hydroxy-2-methylethyl ether (ether of 1,2-propanediol and HMF)

25. bis 5-(hydroxymethyl)furfural 2-methyl-1,2-propane-diyl di-ether (di-ether of 1,2-propanediol and 2 equivalents of HMF)

26. 5-(hydroxymethyl)furfural 3-hydroxypropyl ether (ether of 1,3-propanediol and HMF)

27. bis 5-(hydroxymethyl)furfural 1,3-propane-diyl di-ether (di-ether of 1,3-propanediol and 2 equivalents of HMF)

28. 5-(hydroxymethyl)furfural 2,3-dihydroxypropyl ether (ether of 1,2,3-propanetriol and HMF)

29. bis 5-(hydroxymethyl)furfural 2-hydroxypropaandiyl diether (di-ether of 1,2,3-propanetriol with 2 equivalents of HMF)

30. 5-(hydroxymethyl)furfural 6-hydroxyhexyl ether (ether of 1,6-hexanediol and HMF)

31. bis 5-(hydroxymethyl)furfural 1,6-hexane-diyl di-ether (di-ether of 1,6-hexanediol and 2 equivalents of HMF)

32. ether of 2,5-di(hydroxymethyl)furan en HMF

33. di-ether of 2,5-di(hydroxymethyl)furan and 2 equivalents of HMF

34. ether of 2,5-di(hydroxymethyl)tetrahydrofuran en HMF

35. di-ether of 2,5-di(hydroxymethyl)tetrahydrofuran and 2 equivalents of HMF
